# EUROPEAN PATENT APPLICATION

(11) **EP 0 743 073 A2**
(43) Date of publication of application: **20.11.1996**
(21) Application number: 96303546.4
(22) Date of filing: 17.05.1996
(51) Int. Cl.: A61M 5/32

(54) **Medical needle assembly**

(30) Priority: 19.05.1995 ZA 9504124; 10.08.1995 ZA 9506674
(71) Applicant: Nao, Norman Sipula, Rynfield, Benoni 1514 (ZA)
(72) Inventor: Nao, Norman Sipula, Rynfield, Benoni 1514 (ZA)
(74) Representative: Sturt, Clifford Mark

(57) **Abstract**

A medical needle assembly is provided having an elongate needle and a sheath displaceably mounted on the needle. The assembly further includes a first locking member protruding from the sheath toward the needle and a second complementary locking member protruding from the needle toward the sheath, such that when the second locking member passes the first locking member, the locking members cooperate to lock the needle in a withdrawn position in which a sharp point of the needle is positioned within the sheath.

## Description

THIS INVENTION relates to a medical needle assembly.

In accordance with one aspect of the invention, there is provided a medical needle assembly including
an elongate needle having attachment means at its one end for releasably attaching the needle to a mouth formation of a syringe, and an opposed end adapted to pierce a patient's skin;
a sheath mounted on the needle and having opposed end portions, the sheath at least partially surrounding the needle and extending along its length, the needle being displaceable relative to the sheath between an extended position in which the opposed end of the needle extends outwardly from one end portion of the sheath and a withdrawn position in which the opposed end of the needle is withdrawn into the sheath;
a first locking member intruding from the sheath toward the needle; and
a second complementary locking member protruding from the needle toward the sheath, such that when the second locking member passes the first locking member, the locking members cooperate to lock the needle in its withdrawn position.

It will be understood that the needle and sheath cooperate together to render the needle locked in its withdrawn position, independently of the syringe. Accordingly, the medical needle assembly in accordance with the invention can be mountable on conventional syringes. Furthermore, the medical needle assembly can be integrally formed with a syringe.

The sheath may be generally cylindrical in shape. Conveniently, it is circular-cylindrical in shape.

At least one of the locking members may be resilient. Advantageously, both locking members are resilient.

The second locking member may extend circumferentially around the needle defining an outwardly protruding surface extending generally angularly from the needle in a direction toward the end portion of the sheath through which the opposed end of the needle can be displaced.

The first locking member may extend circumferentially inwardly from an internal surface of the sheath defining an inwardly intruding surface extending generally angularly from the sheath in a direction toward the opposed end portion of the sheath.

The outwardly protruding surface of the second locking member may be generally dome-shaped, the second locking member including a radially extending locking surface intermediate the needle and the outwardly protruding surface, the inwardly intruding surface of the first locking member defining a complementary generally dome-shaped recess and a complementary radially extending locking surface intermediate the internal surface of the sheath and the inwardly intruding surface thereof, such that the dome-shaped surface can pass the complementary dome-shaped recess thereby to cause the radially extending locking surfaces to cooperate and to lock the needle in its withdrawn position.

In use, when the needle is urged into its withdrawn position, the dome-shaped surface of the second locking member abuts against the complimentary shaped recess of the first locking member and, as the needle is displaced into its withdrawn position, the second locking member resiliently passes or rides over the first locking member to be positioned behind the first locking member, the complementary locking surfaces then retaining or locking the needle in its withdrawn position.

Instead, the first locking member may be in the form of a screw-thread, the second locking member being in the form of a complementary screw-thread so as to cause the screw-threads to engage when the sheath and the needle are rotated relative to each other, thereby to lock the needle in its withdrawn position.

The needle may include a breakable portion which protrudes from the sheath when the needle is in its withdrawn position. The breakable portion may have a region of weakness to facilitate the breaking thereof.

The needle assembly may further include a needle cover for covering the needle when in its extended position.

The needle cover may have mounting means for mounting it removably on the sheath.

Advantageously, the needle cover is round-cylindrical in shape. It may have a closed end wall and an opposed open mouth which defines the mounting means.

The invention will now be described, by way of example, with reference to the accompanying diagrammatic drawings, in which:
Figure 1 shows a sectional side view of a medical needle assembly in accordance with the invention;
Figure 2 shows a sectional side view of the medical needle assembly shown in Figure 1, a cover of the medical needle assembly being removed and the needle being in a withdrawn position;
Figure 3 shows a sectional side view of part of the medical needle assembly shown in Figures 1 and 2, the needle assembly being mounted on a syringe;
Figure 4 shows a side view of a needle of the medical needle assembly shown in Figures 1 to 3;
Figure 5 shows a sectional side view of a sheath of the medical needle assembly shown in Figures 1 to 3;
Figure 6 shows a sectional side view of a cover of the medical needle assembly shown in Figures 1 to 3;
Figure 7 shows a three-dimensional view of part of a syringe;
Figure 8 shows a three-dimensional schematic view of another medical needle assembly in accordance with the invention, mounted on a syringe;
Figure 9 shows a schematic sectional side view of the medical needle assembly shown in Figure 8, the medical needle assembly being in an unlocked condition;
Figure 10 shows a schematic sectional side view of the medical needle assembly shown in Figures 8 and 9, the medical needle assembly being in a locked condition;
Figure 11 shows a schematic sectional side view of still another medical needle assembly in accordance with the invention, the medical needle assembly being in an unlocked condition;
Figure 12 shows a schematic sectional side view of the medical needle assembly shown in Figure 11, the medical needle assembly being in an extended position;
Figure 13 shows a schematic sectional side view of the medical needle assembly shown in Figures 11 and 12, the medical needle assembly being in a locked condition;
Figure 14 shows a schematic end view of a sheath of the medical needle assembly shown in Figures 11 to 13, along arrows VII - VII in Figure 13; and
Figure 15 shows an end view of attachment means for attaching the medical needle assembly to a syringe body.

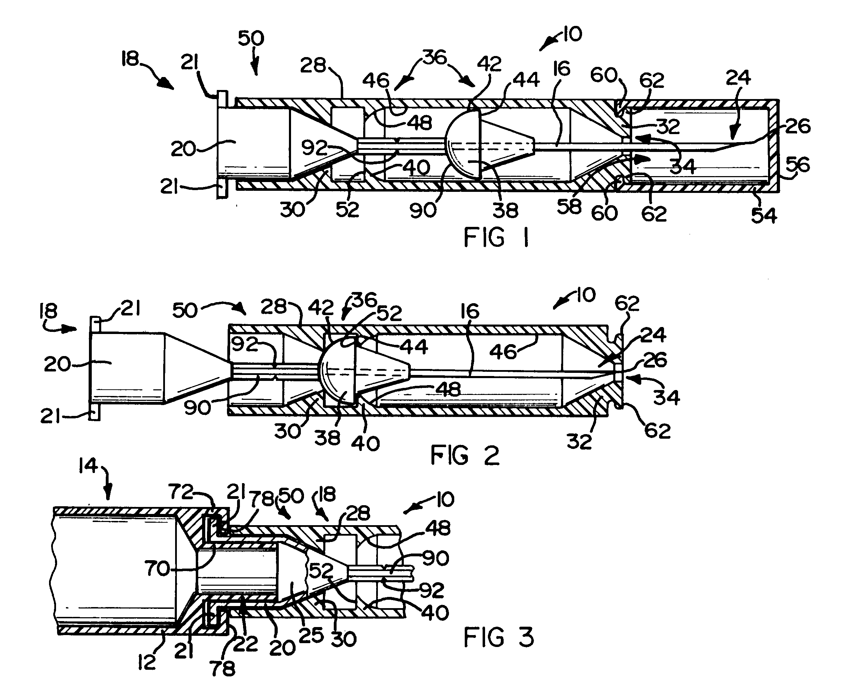

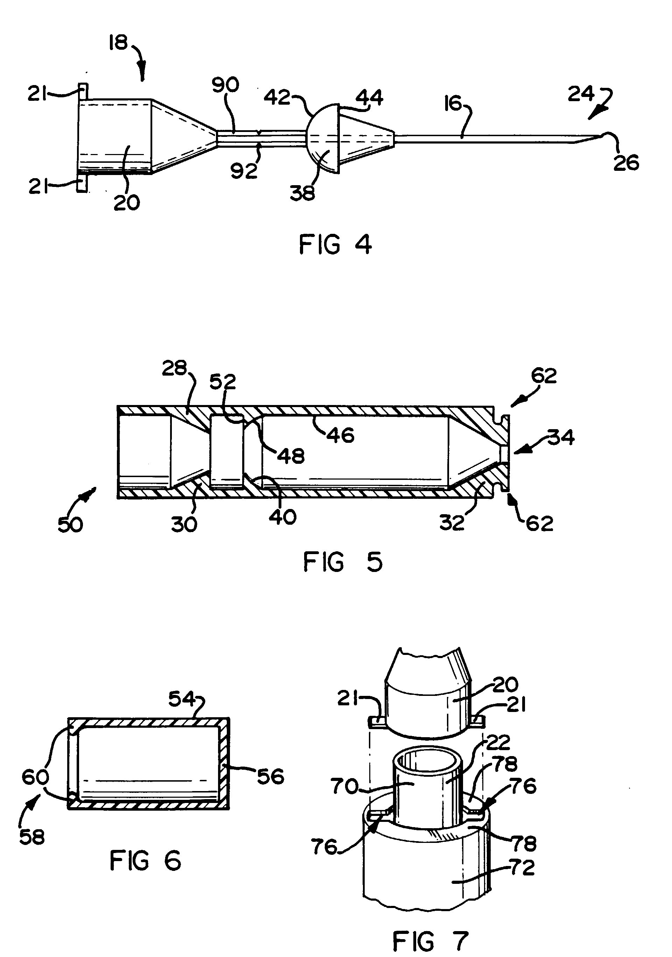

Referring to Figures 1 to 7 of the drawings, a medical needle assembly in accordance with the invention is generally indicated by reference numeral 10. The medical needle assembly 10 is releasably mountable on a syringe body 12 of a syringe 14 (as can best be seen in Figure 3).

The medical needle assembly 10 includes a needle 16. At one end 18 of the needle 16, the needle 16 has attachment means 20 for attaching it releasably to a mouth formation 22 of the syringe 14 for the needle 16 to be in flow communication with the syringe 14. The attachment means 20 is conveniently in the form of a conventional attachment means for attaching a needle to a mouth formation of a syringe and can include a generally conically shaped cavity 25 which snugly fits over the complementarily shaped mouth formation 22 of the syringe. An opposed end 24 of the needle 16 is adapted to pierce a patient's skin, thus, it has a sharp point 26.

The medical needle assembly 10 further includes a sheath 28, displaceably mounted on the needle 16, which surrounds the needle 16 and extends along its length. The sheath 28 is generally circular-cylindrical in shape.

The needle 16 is displaceable relative to the sheath 28 between an extended position in which the opposed end 24 of the needle 16 extends outwardly from the sheath 28 (as can best be seen in Figure 1) and a withdrawn position in which the opposed end 24 of the needle 16 is withdrawn into the sheath 28 (as can best be seen in Figure 2). The needle 16 is held captive within the sheath 28 by means of a stop 30. The stop 30 prevents the needle 16 from being removed from the sheath 28 when in its withdrawn position.

The sheath 28 defines an end wall 32 which has an aperture 34 extending therethrough, through which aperture 34 the opposed end 24 of the needle 16 can pass.

The needle assembly 10 has locking means generally indicated by reference numeral 36. The locking means 36 includes a second locking member 38 protruding outwardly from the needle 16 and a complementary first locking member 40 intruding inwardly from the sheath 28.

The locking member 38 extends circumferentially around the needle 16. It includes an outer surface 42 which extends generally angularly from the needle 16 in the direction of the end wall 32 which has the aperture 34 through which the opposed end 24 of the needle 16 can pass. The outer surface 42 is generally dome-shaped. The locking member 38 further includes a locking surface 44 extending radially intermediate the outer surface 42 and the needle 16.

The locking member 40 extends circumferentially inside the sheath and intrudes from an internal surface 46 of the sheath 28. It has an inwardly intruding or inner surface 48 which extends angularly from the internal surface 46 in the direction of an opposed end 50 of the sheath 28. The inner surface 48 is complementary in shape to the outer surface 42 of the locking member 38 of the needle 16, thus, it is curved. The locking member 40 has a radially extending locking surface 52 intermediate the internal surface 46 of the sheath 28 and the inner surface 48. The locking surface 52 cooperates with the locking surface 44 to retain or lock the needle in a non-releasable locked condition as described in greater detail hereinbelow.

The locking member 40 is resilient to permit the locking member 38 to pass or ride over the locking member 40.

The needle assembly 10 further includes a cover 54. The cover 54 is releasably mountable on the sheath 28 to cover the needle 16 when in its extended position. The cover 54 is round cylindrical in shape and has a closed end 56 and an opposed open end defining a mouth 58.

The cover 54 has engaging formations 60 and the sheath 28 has complementary engaging formations 62. Thus, the cover can be mounted releasably on the sheath 28 by engaging the engaging formations 60 with the engaging formations 62. When the engaging formations 60, 62 are engaged, the sharp point 26 and the portion of the needle 16 which extends from the sheath 28 is covered by the cover 54.

In use, the medical needle assembly 10 is mounted on the syringe body 12 of the syringe 14 in conventional fashion. This can be achieved by inserting the attachment means 20 intermediate an inner and outer cylindrical formation 70, 72 positioned at the mouth formation 22 of the syringe 14. The attachment means 20 can have two opposed ears or tabs 21 which can pass through a slot 76 defined intermediate two flange formations 78 extending inwardly from the outer cylindrical formation 72 toward the inner cylindrical formation 70. Once the ears or tabs 21 have been inserted through the slot 76, the needle assembly 10 can be turned or twisted relative to the syringe 14 for the ears 21 to engage behind the flange formations 78. Instead, the attachment means can be in the form of a hub (not shown) to permit the needle assembly 10 to be attached to a syringe via e.g. a Llewelyn type lock.

Once the needle assembly 10 has been mounted on the syringe 14 in this fashion, the cover 54 can be removed to expose the needle 16. The syringe 14 and needle assembly 10 are then ready for use.

After the needle assembly 10 has been used, the needle assembly 10 can be removed from the syringe 14 by turning the needle assembly relative to the syringe 14 to disengage the ears or tabs 21 from behind the flange formations 78 and passing them through the slot 76. Once removed, the needle 16 can be manually urged by gripping the attachment means 20 and the sheath 28 and pulling the two away from one another. The locking member 38 on the needle 16 is then caused to pass or ride over the locking member 40 of the sheath. Once the locking member 38 has passed over the locking member 40, the locking surface 44 is positioned behind the locking surface 52. The locking surfaces 44, 52 then cooperate to retain or lock the needle 16 in its withdrawn position fish-hook fashion and in a non-releasable locked condition. In its locked condition the needle 16 and its sharp point 26 are positioned internally of the sheath 28, thus the needle 16 is covered by the sheath 28. In this condition, accidental pricking by the needle 16 is inhibited.

Optionally, the needle 16 can include a breakable portion 90 connected to the attachment means 20. The portion 90 can have a region of weakness 92. When the needle is in its locked condition, the portion 90 can be broken at 92, thereby further to inhibit the assembly 10 from being re-used. The breakable portion 90 can be of a suitable synthetic plastics material.

It will be appreciated that the needle 16 has a length which compensates for the length of the sheath 28. The Applicant envisages that the needle 16 can be supplied in different lengths. Thus, where the medical needle assembly is to be used on a child, the portion of the needle 16 extending from the sheath 28 when in its extended condition is of a length which is suitable for injecting the child. Similarly, where the medical needle assembly is to be used on an adult, the portion of the needle 16 extending from the sheath 28 when in its extended condition is of a length which is suitable for injecting the adult. In both cases, when the needle 16 is in its withdrawn and locked condition, its sharp point is covered by the sheath.

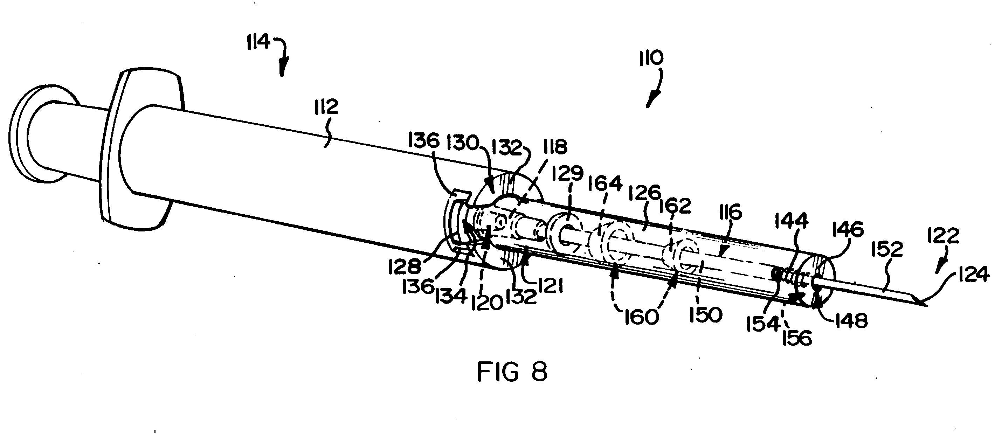

Referring to Figures 8 to 10, another medical needle assembly in accordance with the invention is generally indicated by reference numeral 110. The medical needle assembly 110 is releasably mountable on a syringe body 112 of a syringe 114.

The medical needle assembly 110 includes a needle 116. At one end 121 of the needle 116, the needle 116 has attachment means 118 for attaching it releasably to a mouth formation 120 of the syringe 114 for the needle 116 to be in flow communication with the syringe 114. The attachment means 118 is conveniently in the form of a conventional attachment means for attaching a needle to a mouth formation of a syringe and includes a conically shaped cavity which snugly fits over a complementarily shaped mouth formation of the syringe. An opposed end 122 of the needle 116 is adapted to pierce a patient's skin in use, thus, it has a sharp point 124.

The medical needle assembly 110 further includes a sheath 126 which surrounds the needle 116 and extends along its length. The sheath 126 is generally circular-cylindrical in shape. The sheath 126 defines mounting means in the form of two radially outwardly extending ears or tabs 128 for mounting the sheath 126 releasably on the syringe body 112. The syringe 114 has securing means 130 for releasably securing the sheath 126 in its mounted condition on the syringe 114. The securing means 130 includes two opposed securing formations 132. The securing formations 132 define an intermediate space 134 through which the ears 128 of the sheath 126 can pass. The securing formations 132 define recesses 136. To releasably secure the sheath 126 on the syringe 114 the ears 128 are inserted through the space 134 and the sheath is turned for the ears 128 to be engaged in the recesses 136. Thus, the sheath 126 is securable on the syringe body 112 light-bulb-fashion.

It will be appreciated that when the sheath 126 is secured on the syringe 114 in this fashion, the attachment means 118 is attached to the mouth formation 120 for the needle 116 to be in flow communication with the syringe 114.

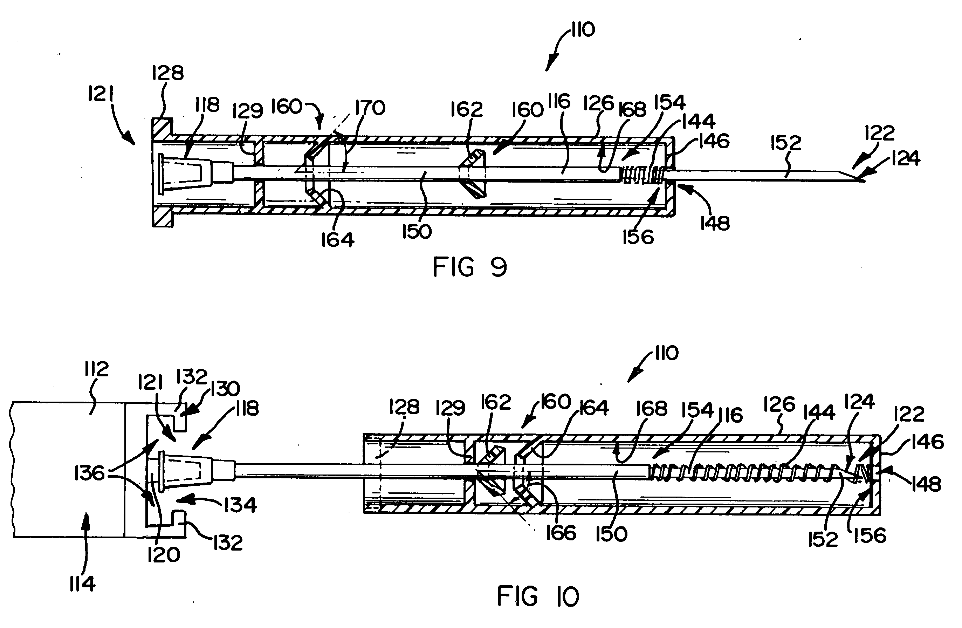

The needle 116 is displaceable relative to the sheath 126 between an extended position in which the opposed end 122 of the needle 116 extends outwardly from the sheath 126 (as can best be seen with reference to Figures 8 and 9) and a withdrawn position in which the opposed end 122 of the needle 116 is withdrawn into the sheath 126 (as can best be seen with reference to Figure 10). The needle 116 is held captive within the sheath 126 by means of a stop. The stop includes a flange formation 129 depending inwardly of the sheath 126 and towards the needle 116.

The medical needle assembly 110 optionally includes urging means in the form of a helical spring 144.

The sheath 126 defines an end wall 146 which has an aperture 148 extending therethrough, through which aperture 148 the opposed end 122 of the needle 116 can pass.

The needle 116 includes a base portion 150 and an injecting portion 152. The base portion 150 has a larger transverse dimension than the injecting portion 152. Thus, the needle 116 has a shoulder 154 extending outwardly from the injecting portion 152. The spring 144 abuts against an inner surface 156 of the end wall 146 of the sheath 126 and against the shoulder 154 of the needle 116. The spring 144 operates under compression and cooperates with the shoulder 154 and inner surface 156 to urge the needle 116 into its withdrawn position.

The needle assembly 110 has locking means generally indicated by reference numeral 160. The locking means 160 includes a locking member 162 protruding radially outwardly from the base portion 150 of the needle 116 and a locking member 164 intruding radially inwardly from the sheath 126.

The locking member 162 extends circumferentially around the base portion 150 of the needle 116. It extends angularly, as indicated at 166, from the base portion 150 in the direction of the end wall 146 which has the aperture 148 through which the opposed end 122 of the needle 116 can pass.

The locking member 164 extends circumferentially around an inner surface 168 of the sheath 126. It extends angularly, as indicated at 170, from the inner surface 168, in the direction of the ears or tabs 128.

The locking members 162, 164 are complimentary to each other. The locking member 162 extends outwardly from the base portion 150 of the needle 116 by a predetermined distance, and similarly the locking member 164 extends inwardly from the inner surface 168 of the sheath 126 by a predetermined distance, such that the locking members 162, 164 overlap each other by a predetermined distance.

The locking members 162, 164 are resilient.

In use, when it desired to mount the medical needle assembly 110 on the syringe 114 the attachment means 118 is attached to the mouth formation 120 of the syringe 114 for the needle to be in flow communication with the syringe 114 (as can best be seen with reference to Figure 10). The sheath 126 is then displaced against the spring 144 and the ears 128 are passed through the space 134. It will be appreciated that the needle 116 is then in its extended position. The sheath 126 is then turned releasably to lock the ears 128 in the recesses 136 for the medical needle assembly to be mounted on the syringe body 112. When the sheath 126 is turned the ears 128 engage with the recesses 136. The syringe and medical needle assembly 110 can then be used in conventional fashion to e.g. inject a patient with medicine. It will be appreciated that the sheath 126 can be turned clockwise or anti-clockwise for the ears 128 to engage in the recesses 136.

After the medical needle assembly 110 has been used, and it is desired to discard the medical needle assembly 110, the medical needle assembly 110 is removed by turning the sheath 126 to permit the ears 128 to disengage from the recesses 136 and to pass through the intermediate space 134. When the sheath 128 is removed from the syringe 114 in this fashion, the spring 144 urges the needle 116 into its withdrawn position until the locking members 162, 164 abut each other. Where a spring 144 is not provided, the needle 116 can be urged manually. It will be appreciated that when the needle 116 is in its withdrawn position, the end 122 of the needle 116 is positioned within the sheath 126 and is then covered by the sheath 126.

The base portion 150 of the needle 116 can then be pulled so that the locking member 162 resiliently passes over the locking member 164. The needle 116 is then in its fully withdrawn position. The locking members 162, 164 then cooperate to prevent the needle 116 from being displaced into its extended position.

The medical needle assembly 110 locks the needle in its fully withdrawn position thus preventing it from being extended and reused.

It will be appreciated that once the needle has been displaced fully into its withdrawn position, the locking members 162, 164 cooperate thereby to lock the needle 116 permanently in its fully withdrawn position. Thus, the medical needle assembly 10 is designed to be used only once and thereafter, if the needle is locked in its fully withdrawn position, the medical needle assembly is prevented from being used again.

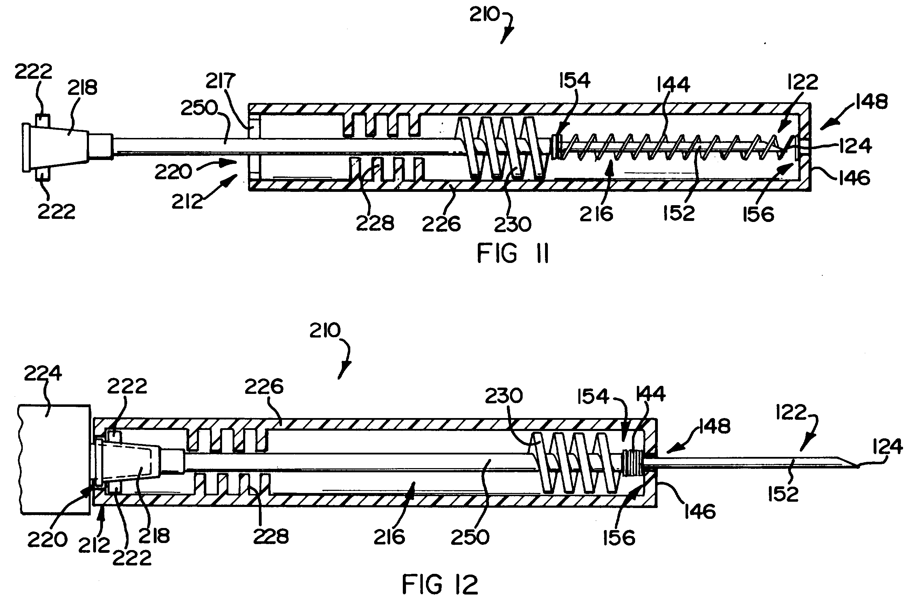

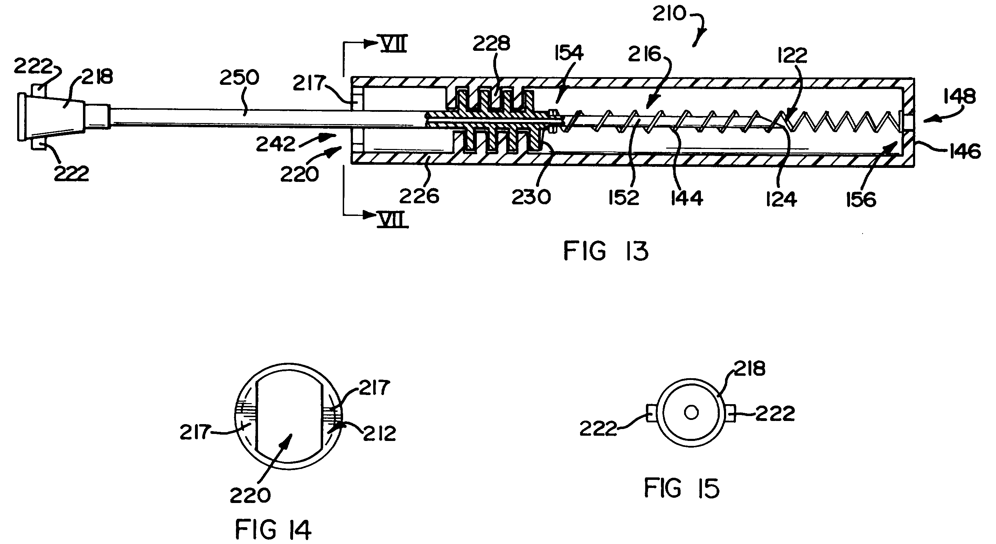

Referring to Figures 11 to 15, another embodiment of the medical needle assembly in accordance with the invention is indicated generally by reference numeral 210. Similar reference numerals as used in Figures 8 to 10 are used in Figure 11 to 15 to designate similar parts unless otherwise stated.

A sheath 226 of the medical needle assembly 210 has securing means 212 for releasably securing it to attachment means 218. The securing means 212 includes a flange formation 217 extending radially inwardly from the sheath 226. The flange formation 217 has a slot 220 extending therethrough.

The attachment means 218 includes two opposed tabs or ears 222 extending radially outwardly. The ears or tabs 222 can pass through the slot 220 of the flange formation 217 when in register therewith.

In use, the attachment means 218 is mounted on a conventional syringe body 224 in conventional fashion. The sheath 226 is then displaced towards the attachment means 218 until its tabs or ears 222 pass through the slot 220 extending through the flange formation 217. The sheath 226 is then twisted relative to a needle 216 of the medical needle assembly 210 for the tabs or ears 222 to be out of register with the slot 220. When out of register, the tabs or ears 222 engage behind the flange formation 217. The needle 216 is then retained in its extended position.

It will be appreciated that the medical needle assembly 110 can have similar securing means 212 as the needle assembly 210. The medical needle assembly 110 can then be mounted on a conventional syringe without any modification to the syringe, such as the securing means 130.

The needle assembly 210 further includes locking means defined by a screw-threaded portion 228 intruding from the sheath 226 and a complementary screw-threaded portion 230 protruding from the needle 216.

The screw-threaded portion 228 extends radially inwardly from the sheath 226 and can be integrally formed therewith. The screw-threaded portion 230 extends radially outwardly from the needle 216 and can be integrally formed with a base portion 250 of the needle 216.

To prevent accidental pricking, the medical needle assembly 210 can be locked so that the end 122 of the needle 216 is in a withdrawn condition by twisting the needle 216 relative to the sheath 226 for the screw-threaded portions 228, 230 to engage. Thus, when the screw-threaded portions 228, 230 are engaged, the medical needle assembly 210 is in a locked condition and accidental pricking is prevented.

The Applicant believes the medical needle assembly 10, 110, 210 as illustrated has several advantages. A major advantage of the medical needle assembly is that when it has been used, the needle can be locked in a position so that it is covered by a sheath. Accordingly, accidental pricking, such as a doctor accidentally pricking himself, or when the medical needle assembly has been discarded, accidental pricking of personnel removing the medical needle assembly from, e.g. a rubbish receptacle, is inhibited. This advantage is of special significance when the medical needle assembly has been used on a patient suffering from a transmittable illness, e.g. AIDS, where accidental pricking could lead to fatal results.

## Claims

1. A medical needle assembly including
an elongate needle having attachment means at its one end for releasably attaching it to a mouth formation of a syringe, and an opposed end adapted to pierce a patient's skin, the medical needle assembly characterised in that it further includes
a sheath operatively mounted on the needle and having opposed end portions, the sheath at least partially surrounding the needle and extending along its length, the needle being displaceable relative to the sheath between an extended position in which the opposed end of the needle extends outwardly from one end portion of the sheath and a withdrawn position in which the opposed end of the needle is withdrawn into the sheath;
a first locking member intruding from the sheath toward the needle; and
a second complementary locking member protruding from the needle toward the sheath, such that when the second locking member passes the first locking member, the locking members cooperate to lock the needle in its withdrawn position.

2. A medical needle assembly as claimed in Claim 1, characterised in that the sheath is generally circular cylindrical in shape.

3. A medical needle assembly as claimed in Claim 1 or Claim 2, characterised in that at least one of the locking members is resilient.

4. A medical needle assembly as claimed in any one of the preceding claims, characterised in that both locking members are resilient.

5. A medical needle assembly as claimed in any one of the preceding claims, characterised in that the second locking member extends circumferentially around the needle and defines an outwardly protruding surface extending generally angularly from the needle in a direction toward the end portion of the sheath through which the opposed end of the needle can pass.

6. A medical needle assembly as claimed in Claim 5, characterised in that the first locking member extends circumferentially inwardly from an internal surface of the sheath and defines an inwardly intruding surface extending generally angularly from the sheath in a direction toward the opposed end portion of the sheath.

7. A medical needle assembly as claimed in Claim 6, characterised in that the outwardly protruding surface of the second locking member is generally dome-shaped, the second locking member including a radially extending locking surface intermediate the needle and the outwardly protruding surface, the inwardly intruding surface of the first locking member defining a complementary generally dome-shaped recess and a complementary radially extending locking surface intermediate the internal surface of the sheath and the inwardly intruding surface thereof, such that the dome-shaped surface can pass the complementary dome-shaped recess thereby to cause the radially extending locking surfaces to cooperate and to lock the needle in its withdrawn position.

8. A medical needle assembly as claimed in any one of Claims 1 to 4, characterised in that the first locking member is in the form of a screw-thread and the second locking member is in the form of a complementary screw-thread so as to cause the screw-threads to engage when the sheath and needle are rotated relative to each other, thereby to lock the needle in its withdrawn position.

9. A medical needle assembly as claimed in any one of the preceding claims, characterised in that the needle includes a breakable portion which protrudes from the sheath when the needle is in its withdrawn position.

10. A medical needle assembly as claimed in Claim 9, characterised in that the breakable portion has a region of weakness to facilitate the breaking thereof.

11. A medical needle assembly as claimed in any one of the preceding claims, characterised in that it includes a needle cover for covering the needle when in its extended position.

12. A medical needle assembly as claimed in Claim 11, characterised in that the needle cover has mounting means for mounting it removably on the sheath.
